# EUROPEAN PATENT SPECIFICATION

(11) **EP 0 756 861 B1**
(45) Date of publication and mention of the grant of the patent: **28.11.2001**
(21) Application number: 96112012.8
(22) Date of filing: 25.07.1996
(51) Int. Cl.: A61K 7/13

(54) **Composition for dyeing of human hair**
Mittel zum Färben von menschlichen Haaren
Composition pour la coloration des cheveux humains

(30) Priority: 31.07.1995 DE 19527978
(43) Date of publication of application: 05.02.1997
(73) Proprietor: Kao Corporation, Chuo-Ku Tokyo 103 (JP)
(72) Inventor: Misu, Daisuke, 64289 Darmstadt (DE)
(74) Representative: Hansen, Bernd, Dr. Dipl.-Chem.

(56) References cited:
- GB-A- 2 168 082
- GB-A- 2 173 515
- GB-A- 2 254 341

## Description

This invention comprises a composition for permanent or semi-permanent dyeing of human hair providing an improved dyeing effect and increased stability.

It is known that hair dyeing compositions are generally classified into two categories, i.e., on the one hand permanent dyeing compositions comprising oxidation dyestuff precursors, which react with oxidizing agents to develop the desired hair coloration depending on the formula chosen; and semi-permanent hair dyeing compositions containing direct dyes which do not need any addition of oxidizing agents to develop their dyeing effect. Obviously, their dyeing results are less durable than those obtained with permanent dyeing compositions.

Dyeing compositions on the basis of direct dyes are usually applied as tinting shampoos, tinting lotions or color setting lotions, optionally also as aerosol foams.

In the case of solutions or lotions, these compositions frequently contain stabilizers with thickening effect, particularly cellulose derivative such as hydroxyethyl cellulose.

The stability and the dyeing effect achieved by the use of these compositions, however, are not satisfactory.

Accordingly, there was a need for hair dyeing compositions on the basis of direct dyes which are stable and provide an improved dyeing result compared with customary compositions. GB-2 168 082 A and GB 2 173 515 A reveal dye compositions for treating hair comprising a cationic surfactant, a direct dye and a liquid vehicle. Examples describe compositions comprising hydroxypropyl Guar, N⁴-(2-hydroxyethyl) -2-nitro-p-phenylene diamine and, in example 4 of GB-A-2 168 082, also 4-nitro-o-phenylene diamine.

The invention is set out in the appended claims.

Due to the addition of hydroxyalkyl Guar derivatives, the dyeing effect of the composition, i.e. its dye absorbing capacity, is essentially increased compared with conventional products; moreover, the composition shows also excellent stability.

The pH-value of the composition according to the invention is within the range from 2 to 10, preferably from 4 to 9, particularly from about 6 to about 8.

A preferred hydroxy-C₂-C₄-alkyl Guar gum derivate, which is used in the hair dyeing composition according to the invention in a proportion of 0.1 % to 7.5 %, preferably from 0.25 % to 5 %, particularly from 0.5 % to 2.5 % by wt., is hydroxypropyl Guar, i.e. the propyleneglycol ether of Guar gum as well as a quaternization product thereof, particularly hydroxypropyl Guar hydroxypropyl trimonium chloride. Further suitable hydroxyalkyl Guar derivates are, e.g., hydroxyethyl Guar, hydroxybutyl Guar and the quaternization products thereof.

Suitable products are on the market under the trade names "Jaguar HP ®", Jaguar C-17 ®", and "Jaguar C-162 ®" as well as "Galactosol ®".

On principle, all direct cationic dyestuffs admitted for this purpose may be used as direct hair dyes; in this respect reference is made to the German Cosmetic Regulations, "Verordnung über kosmetische Mittel (Kosmetik Verordnung)", in its latest version, Annex No. 3.

The stability and absorptive properties of cationic (basic) dyes are particularly enhanced by the addition of Guar gum derivates according to the invention.

Particularly suitable basic (cationic) dyestuffs are

| | |
|---|---|
| Basic Blue 6, | C.I.-No. 51,175; |
| Basic Blue 7, | C.I.-No. 42,595; |
| Basic Blue 9, | C.I.-No. 52,015; |
| Basic Blue 26, | C.I.-No. 44,045; |
| Basic Blue 41, | C.I.-No. 11,154; |
| Basic Blue 99, | C.I.-No. 56,059; |
| Basic Brown 4, | C.I.-No. 21,010; |
| Basic Brown 16, | C.I.-No. 12,250; |
| Basic Brown 17, | C.I.-No. 12,251; |
| Basic Green 1, | C.I.-No. 42,040; |
| Basic Red 2, | C.I.-No. 52,240; |
| Basic Red 22, | C.I.-No. 11,055; |
| Basic Red 76, | C.I.-No. 12,245; |
| Basic Violet 1, | C.I.-No. 42,535; |
| Basic Violet 3, | C.I.-No. 42,555; |
| Basic Violet 10, | C.I.-No. 45,170; |
| Basic Violet 14, | C.I.-No. 42,510; |
| Basic Yellow 57, | C.I.-No. 12,719. |
| Acid Red 18, | C.I. No. 16,255: |
| Acid Red 27, | C.I. No. 16,185; |
| Acid Red 87, | C.I. No. 45,380; |
| Acid Red 92, | C.I. No. 45,410; |
| Acid Violet 43, | C.I. No. 60,730; |
| Acid Yellow 1, | C.I. No. 10,316; |
| Acid Yellow 23, | C.I. No. 19,140; |
| Acid Yellow 3, | C.I. No. 47,005; |
| D&C Brown No.1, | C.I. No. 20,170; |
| D&C Green No.5, | C.I. No. 61,570; |
| D&C Orange No.4, | C.I. No. 15,510; |
| D&C Orange No.10, | C.I. No. 45,425; |
| D&C Orange No.11, | C.I. No. 45,425:1; |
| D&C Red No.21, | C.I. No. 45,380:2; |
| D&C Red No.27, | C.I. No. 45,410:1; |
| D&C Red No.33, | C.I. No. 17,200; |
| D&C Yellow No.7, | C.I. No. 45,350:1; |
| D&C Yellow No.8, | C.I. No. 45,350; |
| FD&C Red No.4, | C.I. No. 14,700; |
| FD&C Yellow No.6, | C.I. No. 15,985. |

The proportion of direct dyes in the compositions according to the invention may be varied from about 0.01% to about 1.5%, preferably 0.05% to 1%, particularly 0.1% to 0.5% by wt. of the total composition.

The hair dyeing composition according to the invention preferably comprises at least one synthetic or natural hair conditioning polymer, preferably in a proportion from about 0.1% to 2.5%, particularly 0.25% to 1.5% by wt. of the total composition. Although any kind of polymers may be used, i.e. nonionic, anionic, amphoteric and cationic polymers, cationic polymers are preferred within the scope of the invention.

Particularly suitable in this respect are the well-known quaternary cellulose derivatives of the type "Polymer JR", quaternized homo- and copolymers of dimethyl diallyl ammonium chloride, being on the market under the trade name "Merquat", quaternary vinyl pyrrolidone copolymers, especially with dialkyl aminoalkyl (meth)acrylates known under the trade name "Gafquat", copolymers from vinyl pyrrolidone and vinyl imidazolinium methochloride offered on the market under the trade name "Luviquat", polyamino-polyamide derivatives, e.g., copolymers of adipic acid dimethyl aminohydroxypropyl diethylene triamine sold under the name "Cartaretine F", as well as also bisquaternary long-chain ammonium compounds of the urea structure described in US Patent 4,157,388 being marketed under the trade name "Mirapol A 15".

In this context, reference is also made to the cation-active polymers described in German Patent Applications Nos. 25 21 960, 28 11 010, 30 44 738, and 32 17 059, and the products listed in European Patent Application No. 337,354, pp.3 to 7. Mixtures of different cationic polymers may also be used.

Nonionic polymers may also be used instead of cationic polymers or combined with those. Suitable nonionic polymers are mainly polyvinyl pyrrolidone homo- and copolymers, particularly polyvinyl pyrrolidone, copolymers of vinyl pyrrolidone and vinyl acetate or terpolymers of vinyl pyrrolidone, vinyl acetate and vinyl propionate, as they are, e.g., sold by BASF under the trade name "Luviskol".

However, (co-)polymers of different acrylic and methacrylic esters, acrylamide and methacrylamide, e.g., polyacrylamide having a molecular weight exceeding 100,000, dimethylhydantoin formaldehyde resins, etc., are also suitable. Of course, mixtures of different nonionic polymers are also applicable.

Suitable anionic polymers within the scope of the invention are vinyl alkyl ether, particularly methyl vinyl ether/maleic acid copolymers, prepared by hydrolysis of vinyl ether/maleic acid anhydride copolymers and sold under the trade name "Gantrez AN or ES". These polymers may be partially esterified, e.g., "Gantrez ES 225", the ethyl ester of an ethyl vinyl ether/maleic acid copolymer or the butyl or isobutyl ester thereof.

Other suitable anionic polymers are particularly copolymers of vinyl acetate/crotonic acid or vinyl acetate/vinyl neodecanoate/crotonic acid of the type "Resyn"; copolymers of sodium acrylate/vinyl alcohol of the type "Hydagen F", sodium polystyrene sulfonate, e.g. "Flexan 130"; copolymers of ethyl acrylate/acrylic acid/N-tert.-butyl acrylamide of the type "Ultrahold"; copolymers of vinyl pyrrolidone/vinyl acetate/itaconic acid, copolymers of acrylic acid/acrylamide or the sodium salts thereof of the type "Reten"; etc.

On principle, all anionic polymers suggested for hair preparations may be used.

As amphoteric polymers which are used either as an alternative to or in admixture with the other polymers, particularly cationic polymers, preferably copolymers of N-octyl acrylamide, (meth)acrylic acid and tert.-butyl aminoethyl methacrylate of the type "Amphomer" are named; as well as copolymers of methacryloyl ethyl betaine and alkyl methacrylates of the type "Yukaformer", e.g., butyl methacrylate copolymer "Yukaformer AM75"; copolymers from monomers bearing carboxylic or sulfo groups, e.g., (meth)acrylic acid and itaconic acid with monomers containing basic groups, particularly amino groups such as mono- and dialkyl aminoalkyl (meth)acrylates or mono and dialkyl aminoalkyl (meth)acrylamides; copolymers of n-octylamide, methyl methacrylate, hydroxypropyl methacrylate, N-tert.-butyl aminoethyl methacrylate and acrylic acid and the copolymers disclosed in US Patent No.3,927,199.

The hair dyeing compositions according to the invention may comprise all additives usual in these preparations, whose type and character depend on the kind of application. These are surfactants, particularly anionic surfactants such as long-chain N-acylamino carboxylic acids and the salts thereof, e.g. N-lauroyl sarcosinate and glutamate, amphoteric surfactants such as betaines, e.g., cocoamidopropyl betaine, as well as nonionic and cationoic surfactants, fats, fatty alcohols, emulsifiers, pH-regulants, solvents and compounding agents, solubilizers, preservatives, perfumes, etc.

The hair dyeing compositions according to the invention are formulated as emulsions, dispersions or solutions and may also be applied as aerosol foams. These compositions and their production are basically known to the expert and need no further explanation.

Particularly preferred are aqueous or aqueous-alcoholic solutions, for instance on the basis of 80% to 95% water and 5% to 20% lower alcohol such as ethanol, n-propanol or isopropyl alcohol.

The following examples describe the composition of preparations according to the invention and show their superiority compared with customary products.

### Examples

| | **No.1 Light-brown** % by wt. | **No.2 Mahogany** % by wt. | **No.3 Mid-blond** % by wt. |
|---|---|---|---|
| Hydroxypropyl Guar | 1.00 | | |
| Guar hydroxypropyl trimonium chloride | | 1.00 | |
| Hydroxypropyl Guar hydroxypropyl trimonium chloride | | | 2.00 |
| Polyquaternium-7 | 0.80 | 0.80 | 0.80 |
| Cocoamidopropyl betaine | 1.00 | 1.00 | 1.00 |
| Dimethicone Copolyol | 0.10 | 0.10 | 0.10 |
| Ethanol | 5.00 | 5.00 | 5.00 |
| Perfume | 0.20 | 0.20 | 0.20 |
| Basic Blue 99 | 0.066 | | |
| Basic Red 76 | | 0.030 | |
| Basic Brown 16 | | | |
| Basic Yellow 57 | | | |
| Basic Brown 17 | 0.050 | 0.200 | 0.036 |
| Disperse Black 9 | 0.004 | | 0.077 |
| Disperse Blue 3 | 0.015 | 0.040 | 0.019 |
| HC Red No. 3 | 0.060 | 0.360 | 0.024 |
| Water ad | 100.00 | 100.00 | 100.00 |

Replacing hydroxypropyl Guar or its quaternization products by the same amount of hydroxyethyl cellulose led to products whose dye absorptive properties, i.e., their coloration power, after 30 minutes processing time at 30°C on average, were 20% to 25% lower than that of the compositions according to the invention.

## Claims

1. Composition for dyeing of human hair, comprising at least one cationic dye in an aqueous or aqueous-alcoholic medium, containing from 0.1 % to 7.5 % by wt., calculated to the total composition, of at least one hydroxy-C₂-C₄-alkyl Guar gum or the quaternary salts thereof, wherein the following compositions are excluded:
| | Weight Percent |
|---|---|
| Hydroxy propyl guar | 0.7 |
| Amodimethicone and Tallotrimonium Chloride and Nonoxynol-10 | 1.0 |
| Hydroxy functional silicone [Dow Corning Silicone DF 11/884] | 0.5 |
| Cetyl alcohol | 1.5 |
| Glycerin | 4.5 |
| N⁴-(2-hydroxy ethyl)-2-nitro-p-phenylene diamine | 0.4 |
| 4-Nitro-o-phenylene diamine | 0.2 |
| Perfume | qs |
| Preservatives | qs |
| Deionised water | qs to 100 |
| | Weight Percent |
|---|---|
| Hydroxy propyl guar | 0.7 |
| Trimethyl silylamodimethicone (and) Octoxynol-40 (and) Isolaureth -6 (and) glycol | 1.0 |
| Hydroxy functional silicone [Dow corning Silicone DF 11/884] | |
| Cetyl alcohol | 1.5 |
| Glycerin | 4.5 |
| N⁴-(2-hydroxy ethyl)-2-nitro-p-phenylene diamine | 1.0 |
| Perfume | qS |
| Preservative | qS |
| Deionised water | qS to 100 |
and
| | Weight Percent |
|---|---|
| Hydroxy propyl guar | 0.7 |
| Amino functional polydimethyl siloxane (emulsified with Polyoxyethylene (10) nonyl phenyl ether) | 1.0 |
| Glycerin | 4.5 |
| N⁴-(2-hydroxyethyl)-2-nitro-p-phenylene diamine | 0.4 |
| 4-Nitro-o-phenylene diamine | 0.2 |
| Perfume | qs |
| Preservatives | qs |
| Deionised water | qs to 100 |

2. Composition according to claim 1, containing 0.25 % to 2.5 % by wt., calculated to the total composition, of at least one hydroxyalkyl Guar gum or the quaternary salts thereof.

3. Composition according to claim 1 or 2, containing as hydroxyalkyl Guar gum hydroxypropyl Guar gum or a quaternary salt thereof.

4. Composition according to one or more of claims 1 to 3, **characterized in that** it has a pH-value between 4 and 9.

5. Composition according to one or more of claims 1 to 4, **characterized in that** it contains from 0.1 % to 2.5 %, calculated to the total composition, of at least one hair conditioning polymer.

6. Use of at least one hydroxy-C₂-C₄-alkyl Guar gum or of the quaternary salts thereof to increase the coloring power of compositions for dyeing and re-dyeing of human hair on the basis of at least one direct cationic hair dyestuff in an aqueous or aqueous-alcoholic medium.

## Patentansprüche

1. Zusammensetzung zum Färben von menschlichen Haaren, enthaltend mindestens einen kationischen Farbstoff in einem wässrigen oder wässrig-alkoholischen Medium, umfassend 0,1 bis 7,5 Gew.-%, berechnet auf die Gesamtzusammensetzung, mindestens eines Hydroxy-C₂-C₄-alkyl-Guar Gums bzw. dessen quarternäre Salze, worin die folgenden Zusammensetzungen ausgeschlossen sind:
| | Gew.% |
|---|---|
| Hydroxypropyl-Guar | 0.7 |
| Amodimethicon und Tallotrimoniumchlorid und Nonoxynol-10 | 1.0 |
| Hydroxy-funktionelles Silicon [Dow Corning Silicone DF 11/884] | 0.5 |
| Cetylalkohol | 1.5 |
| Glycerin | 4.5 |
| N⁴-(2-Hydroxyethyl)-2-nitro-p-phenylendiamin | 0.4 |
| 4-Nitro-o-phenylendiamin | 0.2 |
| Parfum | qs |
| Konservierungsmittel | qs |
| entionisiertes Wasser | qs ad 100 |
| | Gew.% |
|---|---|
| Hydroxylpropyl-Guar | 0.7 |
| Trimethylsilylamodimethicon (und) Octoxynol-40 (und) Isolaureth -6 (und) Glycol | 1.0 |
| Hydroxy-funktionelles Silicon [Dow Corning Silicone DF 11/884] | |
| Cetylalkohol | 1.5 |
| Glycerin | 4.5 |
| N⁴-(2-Hydroxyethyl)-2-nitro-p-phenylendiamin | 1.0 |
| Parfum | qS |
| Konservierungsmittel | qS |
| Entionisiertes Wasser | qS ad 100 |
| | Gew.% |
|---|---|
| Hydroxypropyl-Guar | 0.7 |
| Amino-funktionelles Polydimethylsiloxan (emulgiert mit Polyoxyethylen (10) nonylphenylether) | 1.0 |
| Glycerin | 4.5 |
| N⁴-(2-Hydroxyethyl)-2-nitro-p-phenylendiamin | 0.4 |
| 4-Nitro-o-phenylendiamin | 0.2 |
| Parfum | qs |
| Konservierungsmittel | qs |
| entionisiertes Wasser | qs ad 100 |

2. Zusammensetzung nach Anspruch 1, umfassend 0,25 bis 2,5 Gew.-%, berechnet auf die Gesamtzusammensetzung, mindestens eines Hydrxyalkyl-Guar Gums bzw. dessen quaternäre Salze.

3. Zusammensetzung nach Anspruch 1 oder 2, umfassend als Hydroxyalkyl-Guar Gum Hydroxylpropyl-Guar Gum bzw. ein quaternäres Salz desselben.

4. Zusammensetzung nach einem oder mehreren der Ansprüche 1 bis 3, **dadurch gekennzeichnet, dass** es einen pH-Wert zwischen 4 und 9 aufweist.

5. Zusammensetzung nach einem oder mehreren der Ansprüche 1 bis 4, **dadurch gekennzeichnet, dass** es 0,1 bis 2,5 Gew.-%, berechnet auf die Gesamtzusammensetzung, mindestens eines haarkonditionierenden Polymers enthält.

6. Verwendung mindestens eines Hydroxy-C₂-C₄-alkyl-Guar Gums bzw. dessen quaternären Salzen zur Erhöhung der Farbintensität von Mitteln zum Färben und Nachfärben von menschlichen Haaren auf Basis mindestens eines direktziehenden kationischen Haarfarbstoffs in einem wässrigen oder wässrig-alkoholischen Medium.

## Revendications

1. Composition pour la teinture des cheveux humains, comprenant au moins un colorant cationique dans un milieu à base d'eau ou d'alcohool aqueuse, contenant de 0,1 % à 7,5 % en poids, calculés par rapport à la composition totale, d'au moins une gomme d'hydroxy(alkyle en C₂ à C₄)guar ou de ses sels quaternaires, les compositions suivantes étant exclues :
| | Pourcentage en poids |
|---|---|
| Hydroxypropylguar | 0,7 |
| Amodiméthicone, chlorure de tallotrimonium et nonoxynol-10 | 1,0 |
| Silicone à fonctionnalités hydroxy [silicone Dow Corning DF 11/884] | 0,5 |
| Alcool cétylique | 1,5 |
| Glycérine | 4,5 |
| N⁴-(2-hydroxyéthyl)-2-nitro-p-phénylène diamine | 0,4 |
| 4-nitro-o-phénylène diamine | 0.2 |
| Parfum | qs |
| Agents de conservation | qs |
| Eau déionisée | qs pour 100 |
| | Pourcentage en poids |
|---|---|
| Hydroxypropylguar | 0.7 |
| Triméthylsilylamodiméthicone (et) octoxynol-40 (et) isolaureth-6 (et) glycol | 1.0 |
| Silicone à fonctionnalités hydroxy [silicone Dow Corning DF 11/884] | |
| Alcool cétylique | 1.5 |
| Glycérine | 4,5 |
| N⁴-(2-hydroxyéthyl)-2-nitro-p-phénylène diamine | 1.0 |
| Parfum | qs |
| Agents de conservation | qs |
| Eau déionisée | qs pour 100 |
et
| | Pourcentage en poids |
|---|---|
| Hydroxypropylguar | 0,7 |
| Polydiméthylsiloxane à fonctionnalités amine (émulsifié avec du polyoxyéthylène (10) nonylphényléther) | 1,0 |
| Glycérine | 4.5 |
| N⁴-(2-hydroxyéthyl)-2-nitro-p-phénylène diamine | 0,4 |
| 4-nitro-o-phénylène diamine | 0,2 |
| Parfum | qs |
| Agents de conservation | qs |
| Eau déionisée | qs pour 100 |

2. Composition selon la revendication 1, contenant de 0,25 % à 2,5 % en poids. calculés par rapport à la composition totale, d'au moins une gomme d'hydroxyalkylguar ou de ses sels quaternaires.

3. Composition selon la revendication 1 ou 2, contenant comme gomme d'hydroxyalkylguar une gomme d'hydroxypropylguar ou un sel quaternaire de celle-ci.

4. Composition selon l'une ou plusieurs des revendications 1 à 3, **caractérisée en ce qu'**elle présente un pH dont la valeur est comprise entre 4 et 9.

5. Composition selon l'une ou plusieurs des revendications 1 à 4, **caractérisée en ce qu'**elle contient de 0,1 % à 2,5 %, calculés par rapport à la composition totale, d'au moins un polymère de conditionnement des cheveux.

6. Utilisation d'au moins une gomme d'hydroxy(alkyle en C₂ à C₄)guar ou de ses sels quaternaires pour augmenter le pouvoir colorant de compositions de teinture et de reteinture des cheveux humains à base d'au moins un colorant cationique direct des cheveux dans un milieu à base d'eau ou d'alcohool aqueuse.
